# EUROPEAN PATENT APPLICATION

(11) **EP 4 670 645 A1**
(43) Date of publication of application: **31.12.2025**
(21) Application number: 25184508.7
(22) Date of filing: 23.06.2025
(51) Int. Cl.: A61B 8/06, G01S 15/89, A61B 8/00

(54) **ADAPTIVE CLUTTER FILTERING IN ULTRASOUND COLOR IMAGING**

(30) Priority: 24.06.2024 US 202418751804
(71) Applicant: Siemens Medical Solutions USA, Inc., Malvern, PA 19355 (US)
(72) Inventor: KIM, Jihan, Issaquah, WA, 98029 (US)
(74) Representative: HKW Intellectual Property PartG mbB

(57) **Abstract**

For adaptive clutter filtering (120) in color imaging (140) by an ultrasound scanner (500), artificial intelligence (242) discriminates (110) between types of signals and their corresponding locations. This discrimination (110) may be based on scan data (200) from the beamformer and/or estimates (240) of flow or motion. The wall filter (250) adapts (122) or is programmed (120) to use different frequency response location-by-location based on the discrimination (110).

## Description

### BACKGROUND

The present embodiments relate to ultrasound-based color imaging. Color imaging estimates the velocity, power or energy, and/or variance of motion in a patient from ultrasound return echoes. Color flow imaging estimates for moving fluid. Color tissue imaging estimates for moving tissue.

A wall filter or clutter filter is applied to suppress signals from stationary tissues and other undesired sources. Usually, a high pass filter with a given cutoff is applied based on the assumption that tissues are stationary or not moving. Organ or tissue parts move due to the heartbeat or respiration, so some tissue movement may not be suppressed. Signals from organs with high reflectivity such as bones or muscle interfaces may not be suppressed enough with a wall filter even with the very small movement. Such failure in wall filtering is displayed as flash artifacts or appears incorrectly as normal color, hindering accurate diagnosis, especially in detecting low velocity flow.

Adaptive wall filter techniques address flash or clutter artifacts. For example, a non-linear decomposition-based approach, such as Singular Value Decomposition (SVD), is used for adaptive wall filtering. As another example, a clutter map is generated based on a hand-written algorithm and hand-written parameters for adaptive wall filtering. U.S. patent No. 9,420,997 describes a method using frame-by-frame correlation to adaptively adjust the wall filter cutoff. However, non-linear decomposition and hand-written algorithm approaches require intensive effort and a long time to optimize. Depending on the optimizer's skills and knowledge, the results may widely vary. Different parameter optimization may be needed depending on the imaging application. For example, cardiovascular flow and kidney flow imaging need different optimization. The algorithm may not cover all the different imaging cases.

### SUMMARY

By way of introduction, the preferred embodiments described below include a method, system, computer readable medium, and instructions (computer program) for adaptive clutter filtering in color imaging by an ultrasound scanner. Artificial intelligence discriminates between types of signals and their corresponding locations. This discrimination may be based on scan data from the beamformer and/or estimates of flow or motion. The wall filter adapts or is programmed to use different frequency response location-by-location based on the discrimination. The artificial intelligence operates quickly, allowing real-time discrimination. The artificial intelligence may be trained based on many different imaging applications, so the process may be applied across different imaging cases.

In a first aspect, a method is provided for adaptive clutter filtering in color imaging by an ultrasound scanner. The ultrasound scanner scans a patient. A machine-learned model discriminates a first region from a second region by first and second types of signals represented in scan data from the scanning. A first wall filter is applied for the first region of the first type of signal, and a second wall filter is applied for the second region of the second type of signal. The first wall filter is different than the second wall filter. The ultrasound scanner color images using estimates resulting from the applying of the first and second wall filters.

In a second aspect, a method is provided for adaptive clutter filtering in color imaging by an ultrasound scanner. An artificial intelligence generates a discrimination map discriminating sample locations into multiple categories. Clutter filtering is adapted based on the discrimination map. Color flow imaging uses the clutter filtering as adapted.

In a third aspect, an ultrasound system is provided for color imaging. A transducer and beamformer is provided for scanning a scan region. An image processor is configured to segment, by application of a machine-learned segmentation model, the scan region into at least two classes and adapt settings of a programmable wall filter based on the at least two classes such that different locations of the scan region use different ones of the settings. A Doppler estimator is configured to estimate, from data filtered by the programmable wall filter based on the settings, color values in the scan region. A display is configured to display an image using the color values.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, or non-transitory computer readable storage medium.

The present invention is defined by the following claims, and nothing in this section should be taken as a limitation on those claims. Further aspects and advantages of the invention are discussed below in conjunction with the preferred embodiments.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The components and the figures are not necessarily to scale, emphasis instead being placed upon illustrating the principles of the invention. Moreover, in the figures, like reference numerals designate corresponding parts throughout the different views.
Figure 1 is a flow chart of one embodiment of a method for adaptive wall filtering in color imaging by an ultrasound scanner;
Figure 2 is a flow chart of another embodiment of the method for adaptive wall filtering;
Figure 3 illustrates different wall filtering by location based on a discrimination map;
Figure 4 illustrates example color flow imaging; and
Figure 5 is a block diagram of one embodiment of a system for color imaging with adaptive clutter filtering.

### DETAILED DESCRIPTION OF THE DRAWINGS AND PRESENTLY PREFERRED EMBODIMENTS

A pre-trained semantic segmentation deep network or another artificial intelligence generates a discrimination map, which discriminates the input data pixels into multiple categories. For example, three categories are signal, flash/clutter, and background noise. The deep network or other artificial intelligence classifies the content or signals represented at each signal for selection of the appropriate wall filter. A set of wall filters are applied pixel-by-pixel to the data based on the discrimination map. The wall filtering adapts by selection of the appropriate wall filter for a given pixel.

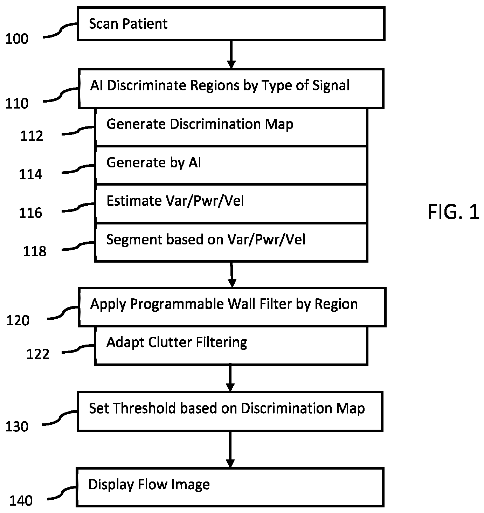

Figure 1 shows one implementation of a method for adaptive clutter filtering in color imaging by an ultrasound scanner. Color or color flow is used to indicate spatial motion imaging, such as fluid or tissue motion. "Color" is used to distinguish from spectral Doppler imaging, where the power spectrum for a range gate is estimated. For color imaging, the estimated flow for each location is mapped to color for display, providing a spatial representation of motion in a scan region. The color "flow" data may not be of fluid (e.g., may be of tissue motion) and/or may not represent color (e.g., may be a scalar). Doppler, color, or flow imaging modes provide color imaging.

The method of Figure 1 provides for adaptive wall filtering in color imaging. Artificial intelligence discriminates types of signals, such as classifying into two, three, or more types, by location. This discrimination provides locations or segments. The wall filtering adapts by applying frequency response appropriate location-by-location based on the discrimination map.

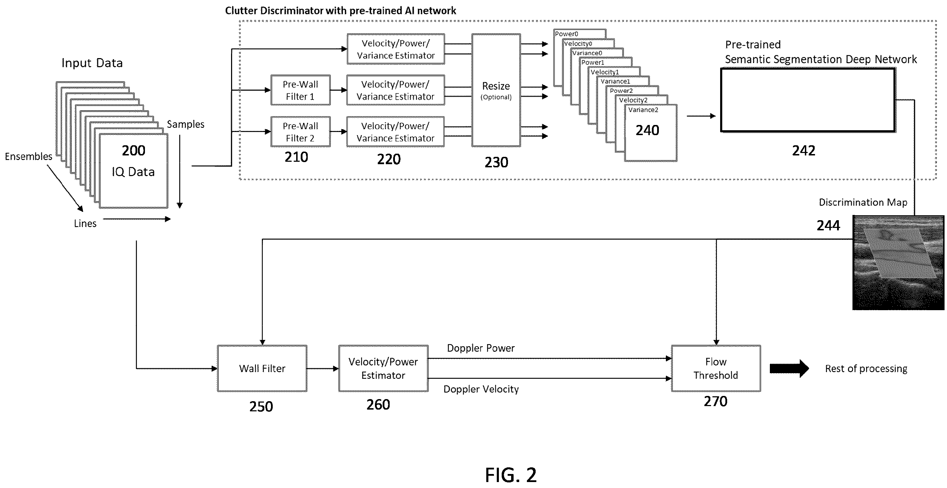

Figure 2 shows a flow chart of another implementation of the method for adaptive wall filtering. An approach using multiple estimates of velocity, variance, and/or power for input to the artificial intelligence to discriminate is shown. A flow threshold also adapts based on the discrimination map in this approach.

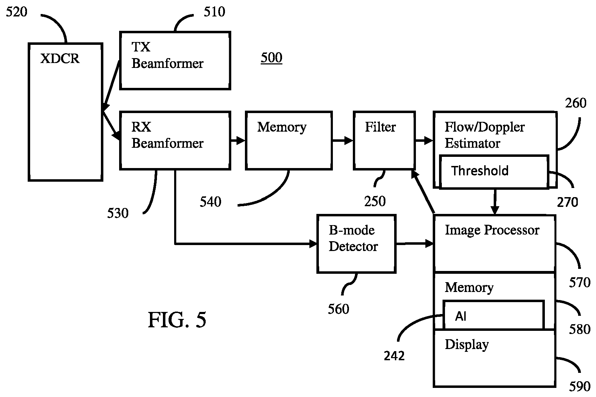

The methods of Figures 1 and 2 are performed by the ultrasound imaging system 500 of Figure 5, the image processor 570, or a different system and/or processor. For example, the ultrasound imaging system 500 performs the acts. As another example, the image processor 570 discriminates and controls the wall filter and/or threshold. The Doppler estimator 260 and a wall filter 250 perform color flow imaging using a beamformer 510, 530 and transducer 520 of the ultrasound scanner. A display 590 is used to display a color image after mapping motion scalar values from the Doppler estimator to color values. A scan converter, graphics memory, temporal filter, and/or other components of an ultrasound scanner may be used for any of the acts.

The acts of Figures 1 and 2 are performed in the order shown or a different order. For example, act 100 is repeated as part of act 140. Acts 112-118 may be performed in any order or simultaneously. All the acts may be repeated for on-going or continuous scanning and color imaging.

Additional, different, or fewer acts than shown in Figures 1 or 2 may be used. For example, act 130 is not provided. As another example, acts 110 and 120 are provided for use with any other acts, including or not including acts 100 and 140. In yet another example, acts for user adjustment, original setting, or manual control over the same or different color imaging parameters are provided.

In act 100, the ultrasound scanner scans a patient. Various locations within a scan region of the patient are scanned with ultrasound. In one embodiment using an ultrasound system, a field of view in a patient is scanned in real-time, providing images while scanning. The scanned region is an interior of an object, such as the patient. The scan is of a volume, plane, or line region. Scanning a plane provides data representing different locations or samples of the plane. The data representing the region is formed from spatial sampling of the object. The spatial samples are for locations distributed in an acoustic sampling grid. Samples may be converted to represent locations in a display format or grid.

The region for the color scan is a region of interest smaller than a field of view or for the entire field of view. The ultrasound system may scan the field of view using B-mode imaging, a combination of B-mode imaging and color flow imaging, or other modes of imaging. The color region is a sub-set of the B-mode field of view. The user or a processor determines the region of interest in which color flow scanning occurs. Alternatively, the color flow region is the full field of view.

For B-mode scanning, the scanning is configured to scan the field of view. For color flow scanning, scan lines in the region of interest are sampled multiple times. The complete region of interest is scanned multiple times in sequence. Scanning at different times in sequence acquires spatial samples associated with motion. Any now known or later developed pulse sequences and/or scan formats may be used for B-mode and color flow scanning. A sequence (flow sample count) of at least two transmissions is provided along each scan line for color flow imaging. For example, the flow sample count is 10-20, resulting in 10-20 samples for each location. Any pulse repetition frequency (i.e., rate of sampling for a location), flow sample count (i.e., number of samples for a location or used to estimate), and pulse repetition interval (i.e., time between each sample acquisition for a location) may be used. Only one transmission along each line is needed for B-mode imaging of the field of view for a given period.

The echo responses to the transmissions or return samples are used to determine intensity for B-mode scanning and estimate velocity, energy (power), and/or variance at a given time for color flow (Doppler) imaging. The transmissions along one line(s) may be interleaved with transmissions along another line(s). With or without interleaving, the spatial samples for a given time are acquired using transmissions from different times. The estimates from different scan lines may be acquired sequentially, but rapidly enough to represent a same time from a user perspective. Multiple scans are performed to acquire estimates for different times.

In alternative embodiments, the return samples (e.g., B-mode data and/or color flow data) are acquired by transfer over a network and/or loading from memory. Data previously acquired by scanning is acquired.

The samples are signals from the patient and/or artifacts. Different locations may have different signal sources. For example, signals from moving and/or non-moving tissue may be background or noise signals for color imaging of fluid flow. Signals representing clutter or flash due to processing by the ultrasound scanner may occur. Signals from the desired moving fluid or tissue may occur for other locations.

In act 110, an image processor, applying a machine-learned model, discriminates different regions by the types of signals represented in the scan data from the scanning. In response to input, the machine-learned model outputs a discrimination map 244 in act 112. The discrimination map 244 is a classification or segmentation location-by-location. The machine-learned model discriminates in act 114 in response to input of data, such as scan data or data derived from the scan data.

Different regions are segmented. The regions of each type of signal may be contiguous or non-contiguous. The discrimination is location-by-location, such as scan data or beamformer sample locations in the region of interest or pixel-by-pixel for display or image sample locations. For the classes used (e.g., background noise, flash and/or clutter, and desired signal (e.g., from moving tissue or fluid)), the machine-learned model indicates to which class each location belongs. In act 112, the discrimination map 244 is generated by the machine-leaned model (e.g., artificial intelligence) to discriminate between types of signals for the different locations.

In one approach, three classes are used for discrimination. As a result, the region of interest samples are segmented into three different regions. For example, locations of signal from desired fluid or moving tissue are identified; locations of signal from flash and/or clutter are identified; and locations of signals from background noise are identified. Two classes may be used, such as desired signals and undesired signals. More than three classes may be used, such as separately classifying flash and clutter, separately classifying moving and non-moving tissue, separately classifying desired movement (e.g., speed of movement) from undesired movement, and/or classifying magnitude of membership (e.g., how likely or to what level of background noise verses desired signal). The discrimination map 244 is generated to distinguish between the sample locations with desired (e.g., flow) signal, the sample locations with clutter and/or flash, and the sample locations with background noise.

Any segmentation may be used. Rather than segmentation hand-coded for a particular application or generalized to multiple applications without being optimized for any of them, the machine-learned model 242 segments. The machine-learned model 242, through previous training, was taught to discriminate in different imaging cases, such as the same machine-learned model 242 being used for heart, liver, thyroid, and/or other color imaging scenarios.

In some embodiments, the machine-learned model 242 is an artificial intelligence. Learnable parameters of the model 242 are learned by optimization using training data. Data from many different ultrasound color imaging examinations of patients are collected. One or more experts may distinguish between different types of signals or segment in these samples of imaging. Alternatively, simulation or other segmentation that may operate well but not rapidly enough is used. The sources of accurate discrimination provide a ground truth (what the discrimination map should look like) given the input examples of the training data. By optimization, the values of the learnable parameters of the model 242 are learned in a training phase. Once trained, the machine-learned model 242 may be applied for use with a patient to generate the discrimination map using the model with the learned values for the learnable parameters.

By using training data from two or more types of ultrasound color imaging examinations (e.g., heart and liver), the machine-learned model 242 was trained to discriminate in different imaging scenarios. The pre-trained model 242 is capable of discriminating input data from various imaging cases since the model 242 is trained on massive data from different imaging scenarios. The same model 242 may be used for any of various imaging applications, so one model 242 on the ultrasound scanner or server may be used for various patients.

Since deep artificial intelligence networks are powerful and versatile enough to accommodate different color flow imaging scenarios, the artificial intelligence-powered adaptive filtering may outperform conventional adaptive filters in terms of clutter suppression and signal preservation. As the model 242 is trained with a lot of data from different imaging scenarios, the model 242 will show more consistent results with different imaging scenarios. The optimization effort and time for development may be significantly reduced since the human optimizer's skill and knowledge will not be the important factor of performance of the artificial intelligence-powered adaptive wall filter.

In some embodiments, the machine-learned model 242 is a neural network, such as a deep learning network. Fully connected, convolutional, and/or other types of neural networks may be used. Where data representing different spatial locations is input with a spatial map output, the model 242 is an image-to-image model in one approach. For example, a U-Net, Link-Net, encoder-decoder, transformer, or other neural network is used. In one approach, a semantic segmentation deep network is used. The neural network outputs class membership by location. The pre-trained semantic segmentation deep network generates the discrimination map 244, which discriminates the input data pixels into multiple categories. Alternatively, a neural network that receives spatial input to output a class for a given location is used. Where data for a given location is input to discriminate, other networks may be used, such as a fully connected neural network or a long-term short term network.

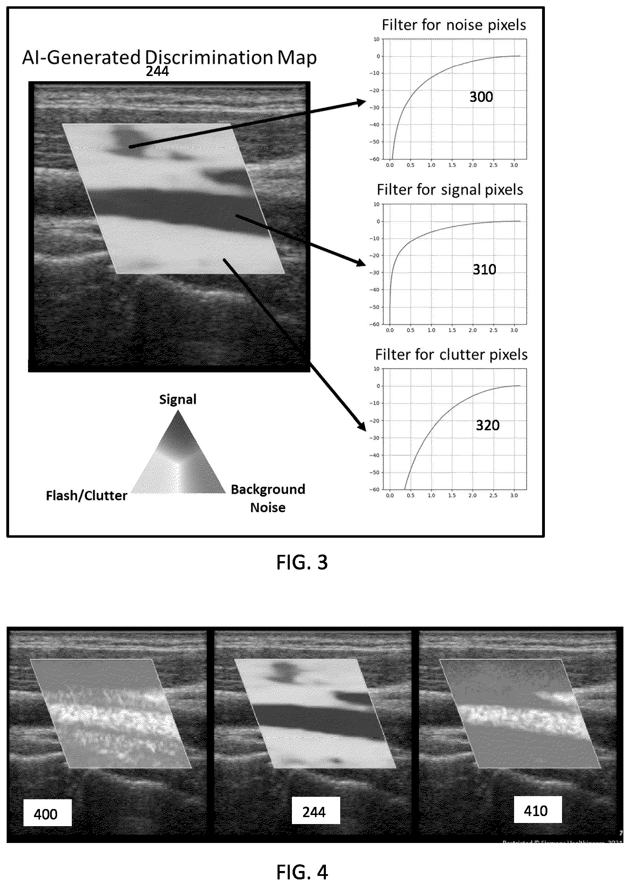

Discriminating data into a few (e.g., three or fewer) classes accurately is a comparably easy task for modern deep networks. As an example, Figure 3 shows an artificial intelligence generated discrimination map 244 with three classes color coded below the map 244 as signal, flash/clutter, and background noise for a region of interest. This discrimination map 244 of Figure 3 is generated based on a deep neural network, such as an off-the-self image-to-image network formed by an encoder and decoder. By using the artificial intelligence to discriminate, the discrimination may consume smaller graphic processing unit (GPU) or central processing unit (CPU) resources and run fast enough to operate at the color imaging frame rate (e.g., 10 Hz, 20 Hz, or faster).

The input to the machine-learned model 242 is scan data. For example, beamformed samples are input. The beamformed samples are prior to estimation of velocity, variance, and power. Beamformed samples may be formatted as in-phase and quadrature (IQ) format (IQ data 200) or another format. All samples for a flow sample count or a sampling of the samples are input.

Figure 2 shows an alternative approach. The artificial intelligence (e.g., model 242) generates the discrimination map 244 in response to input of estimates 240 of velocity, variance, and/or power in act 116 (see Figure 1). In one implementation, an estimator 220 estimates the velocity, variance, and/or power from IQ data 200 without wall filtering 210 or with wall filtering 210. In another implementation, two or more different levels of wall filtering are applied. Figure 2 shows an implementation with one set of estimates being without wall filtering 210 and two other sets of estimates using two other wall filtering 210. Two or more (e.g., three in Figure 2) versions of estimates 240 (e.g., using different levels or wall filtering 210) are generated by the estimator 220. The machine-learned model 242 includes input channels for each set of estimates (e.g., nine channels for three versions of velocity, variance, and power estimates 240). A spatial distribution of estimates 240 are input, providing different versions of the estimates 240 for each location.

The machine-learned model 242 may have input channels restricted to a certain format or size. At 230, the estimates are resized (e.g., down or up sampled) or reformatted for input to the machine-learned model 242.

In response to the input of the estimates 240, the machine-learned model 242 outputs the discrimination map 244. The machine-learned model 242 segments in act 118 (see Figure 1) based on the input variance, velocity, and/or power for one or more versions of the estimates 240.

The resulting discrimination map 244 is used to configure the wall filter 250 and/or threshold 270. When estimating the power, velocity, and/or variance by the estimator 260 for imaging from the scan data 200, the wall filter 250 and/or threshold 270 adapt location-by-location. One or more characteristics or settings of the ultrasound scanner are configured differently for processing of data for different locations. For example, the frequency response and/or cut-off frequency (e.g., 3 dB down) of the wall filter 250 is different for different types of signal.

Figure 3 shows an example. The frequency response 300 for the locations with background noise has a relatively moderate cut-off frequency. The frequency response 310 for the desired signals has a relatively moderate cut-off frequency (e.g., 0.2 at -6 dB of the Nyquist frequency) but with a more aggressive corner. The frequency response 320 for the flash and/or clutter signals has a higher cut-off frequency (e.g., 0.3 at -6 dB of the Nyquist frequency) and a flatter corner. Signal pixels are filtered with moderate cutoff high pass filters to preserve flow signals, back ground noise pixels are filtered with higher cutoff to suppress such noises, and flash/clutter pixels are filtered with a relatively highest cutoff to suppress flash/clutter artifacts. The wall filter 250 is programmed to provide the desired frequency response, so operates differently on different locations depending on the classification from the discrimination map 244.

The wall filter 250 is reprogrammed location-by-location. Alternatively, the wall filter 250 is programmed for one type of signal and all locations for that type are filtered, then the wall filter 250 is reprogrammed for another type of signal and all locations for that type and filtered. Interleaving of programming for different signals may be used. In another alternative, separate wall filters 250 implement the programmable wall filter 250. Each separate wall filter 250 operates on a specific type of signal, so the programming is the selective application of the different wall filters 250.

A set of frequency responses (e.g., settings for a programmable wall filter) is stored. The set includes one for each type of signal. Alternatively, multiple frequency responses are provided for each type of signal. The discrimination map may have greater refinement (e.g., multiple levels) for each type, and/or the frequency response to use for a given type is based on the imaging application or scenario. A controller programs the wall filter 250 based on the selected frequency responses for filtering location-by-location based on the discrimination map 244.

In act 120, the ultrasound scanner applies the wall filter 250. The wall filter 250 programmed for one type of signal is applied to the data from locations in the region of that type of signal. The wall filter 250 programmed for another type of signal is applied to the data from locations in the region of that type of signal. This process may be repeated for additional types of signals. Different wall filters 250, either through programming or selecting different filters, are applied separately to data for different types of signals.

The wall filter 250 is applied to the scan data. Beamformed data (e.g., IQ data 200) prior to estimation by the Doppler estimator 260 is filtered. The received spatial samples are clutter filtered. The clutter filter 250 passes frequencies associated with fluid and not tissue motion or with tissue motion and not fluid. The clutter filtering is of signals in a pulse sequence for estimating motion at a given time (e.g., samples of a flow sample count). A given signal may be used for estimates representing different times, such as associated with a moving window for clutter filtering and estimation. Different filter outputs are used to estimate motion for a location at different times.

The scan data used to discriminate is filtered for imaging. Alternatively, or additionally, later acquired scan data is filtered based on the discrimination from earlier acquired scan data.

In act 122, a set of pixel-by-pixel adaptive wall filters 250 is applied to the data based on the discrimination map 244. The clutter filtering for color imaging (i.e., color Doppler imaging, color flow imaging, or color tissue movement imaging) adapts based on the discrimination map. The different high pass frequency responses selected for the discriminated types of signals (e.g., flow (desired) signal, clutter and/or flash, and background noise) are applied by location. For example, signal pixels are filtered with the frequency response 310 to preserve flow signals, back ground noise pixels are filtered with the frequency response 300 to suppress such noises, and flash/clutter pixels are filtered with the frequency response 320 to suppress flash/clutter artifacts. In this example, the desired signal wall filter 250 has a lowest cutoff frequency relative to the desired signal, flash/clutter, and background noise wall filters 250; the flash/clutter wall filter 250 has a highest cutoff frequency relative to the desired signal, flash/clutter, and background noise wall filters 250, and the background noise wall filter 250 has a cutoff frequency between the highest and lowest cutoff frequencies relative to the desired signal, flash/clutter, and background noise wall filters 250. Other relative arrangements may be used. Other characteristics (e.g., slope or shape) of the frequency response may vary by type of signal.

The filtered data is provided to the estimator 260. The estimator 260 estimates the velocity, variance, and/or power (e.g., energy) using Doppler processing. For each location at a given time or period, the velocity, variance, and/or power is estimated. In the example of Figure 2, the velocity and power are estimated.

Color data (estimates) are generated from the filtered samples. Doppler processing, such as autocorrelation, may be used. In other embodiments, temporal correlation may be used. Another process may be used to estimate the color data. Color Doppler parameter values (e.g., velocity, energy, or variance values) are estimated from the spatial samples acquired at different times. The change in frequency (e.g., Doppler shift) between two samples for the same location at different times indicates the velocity. A sequence (flow sample count) of two or more samples may be used to estimate the color Doppler parameter values. Estimates are formed for different groupings of received signals, such as separate or independent groupings or overlapping groupings. The estimates for each grouping represent the spatial location at a given time.

The estimation is performed for the different sampled spatial locations. For example, velocities for the different locations in a plane are estimated from echoes responsive to the scanning. Multiple frames of color data may be acquired to represent the region of interest at different times, respectively.

The estimates may be thresholded. The controller or another processor sets the threshold for the estimates. To determine whether to display the estimate for a given location, the velocity, variance, and/or power are compared to a threshold. For example, a low velocity threshold is applied. Velocities below the threshold are removed or set to another value, such as zero. As another example, where the energy is below a threshold, the velocity value for the same spatial location is removed or set to another value, such as zero. In other examples, a velocity threshold is applied to determine whether to display velocity and/or power, and/or a power threshold is applied to determine whether to display velocity and/or power. Any combination of one or more thresholds may be used. Alternatively, the estimated velocities are used without thresholding.

In act 130 of Figure 1, the threshold or thresholds vary by location. Locations in a region for one type of signal are compared to a different threshold magnitude than other locations. The threshold is set by region, location, and/or type of signal. The color threshold adapts based on the discrimination map 244 generated by deep network 242. For example, flash/clutter and background noise pixels have relatively higher power and/or velocity thresholds while the thresholds for signal pixels are relatively lower to preserve signals. Other relative thresholds may be used.

The threshold is implemented by a processor 270 or other programmable or selectable hardware. The processor 270 may remove the estimates for some locations based on the threshold. The thresholds more likely pass desired signal and remove undesired signals (e.g., background noise, clutter, and flash). The resulting estimates are output for any further processing, such as spatial filtering, temporal filtering, scan conversion, and/or mapping to colors.

In act 140, a color image is displayed. The ultrasound scanner color images using the estimates resulting from the application of the wall filter 250 (e.g., application of the different wall filters) and/or thresholding. The color flow imaging uses the clutter filtering as adapted with the discrimination map 244. The color image is formed from estimates of the scan data for the different regions or locations. The estimates are formed from the adaptive wall filtering. The adaptive wall filtering leads to estimates more likely to contain desired signal and less likely to contain background noise and clutter/flash. The different frequency responses and/or adaptive thresholding more likely result in accurate color imaging of moving fluid or moving tissue without undesired information.

Figure 4 shows an example. The carotid artery of a patient is scanned. The artificial intelligence generates the discrimination map 244 using the approach of Figure 2. Non-adapted or conventional wall filtering is used to generate the color image 400. Adapting the wall filter based on discriminating between desired signal, background noise, and flash/clutter (see Figure 3) is used for the color image 410. There wall filters and/or corresponding frequency responses are used to adapt to the three types of signals. The color image 410 using the adaptive wall filtering has less color noise so the artery is more definitively represented than in the color image 400.

Where the artificial intelligence (e.g., machine-learned model 242) is trained to discriminate in different imaging applications or scenarios, the adaptation of clutter filtering operates in the different imaging applications or scenarios. The color imaging for one of the various imaging applications benefits from the adaptive clutter filtering. This same benefit is provided for any of the various imaging applications for which the machine-learned model 242 was trained. The same adaptive clutter filtering approach and/or machine-learned model 242 may be used in any of various imaging applications (e.g., carotid, liver, heart, cardiac, thyroid, or another color imaging application).

The color image 410 is displayed. The ultrasound system processes the frame of color data to create the image 410. Spatial filtering, temporal filtering, scan conversion, or another image processing is performed. The scalar values are mapped to display values, such as mapping to color values using a velocity scale. The resulting image is buffered for display. The display values are provided from the buffer to the display.

Color flow (e.g., Doppler energy or Doppler velocity), Doppler tissue motion, or other motion image is generated. The image may include other information. For example, the image is an overlay of the color data on B-mode data. For non-tissue locations or locations associated with sufficient flow, the color flow data (e.g., velocities) are used to determine a color to display. For tissue locations or low/no flow locations, the B-mode data is used.

Figure 5 shows one embodiment of a system 500 for color imaging. The system 500 implements the method of Figure 1, the method of Figure 2, or another method. By using segmentation by a machine-learned segmentation model, clutter filtering and/or thresholding may be adapted to the type of signal.

The system 500 includes a transmit beamformer 510, a transducer 520, a receive beamformer 530, a memory 540, a filter 250, a flow estimator 260, another memory 580, an image processor 570, a display 590, and a B-mode detector 560. Additional, different, or fewer components may be provided. For example, the flow estimator 260 and image processor 570 are provided without the front-end components, such as the transmit and receive beamformers 510, 530. In yet another example, the memories 540 and 580 are one component. As another example, the processor 570 implements the filter 250, estimator 260, and/or detector 560. The filter 250 may be used as one or more of the filters 210 or separate filters are provided. The estimator 260 may be used as one or more of the estimators 220 or separate estimators are provided.

In some embodiments, the system 500 is a medical diagnostic ultrasound system or scanner. In alternative embodiments, the system 500 is or includes a computer or workstation. In yet other embodiments, the flow estimator 260 is part of a medical diagnostic ultrasound system or other medical imaging system, and the image processor 570 is part of a separate workstation or remote system.

The transducer 520 is an array of a plurality of elements. The elements are piezoelectric or capacitive membrane elements. The transducer elements transduce between acoustic and electric energies. The transducer 520 connects with the transmit beamformer 510 and the receive beamformer 530 through a transmit/receive switch, but separate connections may be used in other embodiments.

The transmit and receive beamformers 510, 530 are a beamformer for scanning a region of the patient with the transducer 520. The transmit beamformer 510, using the transducer 520, transmits one or more beams to scan a region. The receive beamformer 530 samples the receive beams at different depths. Sampling the same locations at different times obtains a sequence for flow estimation.

For B-mode scanning, the intensity of the echo for each location is determined by the B-mode detector 560. The field of view is scanned by sampling each location once to provide a frame of B-mode data for a given time.

For imaging motion, such as tissue motion or fluid motion, multiple transmissions and corresponding receptions are performed for each of a plurality of spatial locations. Phase changes between the different receive events for each given location indicate the velocity of the tissue or fluid. A velocity sample group corresponds to multiple transmissions for each of a plurality of scan lines. The number of times a spatial location, such as a scan line, scanned within a velocity sample group is the velocity or flow sample count. The transmissions for different scan lines, different velocity sample groupings, or different types of imaging may be interleaved. The amount of time between transmissions to a same scan line within the velocity sample count is the pulse repetition interval. The pulse repetition interval establishes the pulse repetition frequency or vice versa.

The memory 540 is video random-access memory, random access memory, removable media (e.g., diskette or compact disc), hard drive, database, corner turning memory, buffer, or other memory device for storing data or video information. In some embodiments, the memory 540 is a corner turning memory of a motion parameter estimation path. The memory 540 is configured to store signals responsive to multiple transmissions along a same scan line. The memory 540 is configured to store ultrasound data formatted in an acoustic grid, a Cartesian grid, both a Cartesian coordinate grid and an acoustic grid, or ultrasound data representing a volume in a 3D grid. The return samples of the flow sample count for each of a plurality of locations are stored.

The wall filter 250 is a clutter filter, finite impulse response filter, infinite impulse response filter, analog filter, digital filter, combinations thereof or other now known or later developed filter. In some embodiments, the filter 250 includes a mixer to shift signals to baseband and a programmable low pass filter response for removing or minimizing information at frequencies away from the baseband. In other embodiments, the filter 250 is a low pass, high pass, or band pass filter.

The wall filter 250 is programmable. The frequency response of the wall filter 250 is changed by programming. The programming may be selection of a wall filter from a set. The programming may be setting configurable parameters of the wall filter 250. The programming may be selection of a desired frequency response to be implemented by a processor 570.

The filter 250 may be programmed, such as altering operation as a function of signal feedback or other adaptive process. In yet other embodiments, the memory 540 and/or the filter 250 are part of the flow estimator 260.

The image processor 570 is a digital signal processor, a general processor, an application specific integrated circuit, field programmable gate array, control processor, central processing unit, digital circuitry, analog circuitry, graphics processing unit, combinations thereof, or another now known or later developed device for adapting the clutter filtering and/or thresholding. The image processor 570 operates pursuant to instruction provided in the memory 580 or a different memory. Additional or multiple processors may be used or form the processor 570. The image processor 570 is configured by software, firmware, and/or hardware.

The image processor 570 receives B-mode and/or flow data from the B-mode detector, flow estimator 260, the memory 580, and/or another source. Alternatively, or additionally, the image processor 570 receives beamformed data from the receive beamformer 530 or the memory 540.

Using the received data, the image processor 570 is configured to segment the scan region into at least two classes and adapt settings of the programmable wall filter 250 based on the at least two classes such that different locations of the scan region use different ones of the settings. All of the locations are classified. The image processor 570 applies the machine-learned segmentation model (artificial intelligence) 242. The beamformed data (e.g., IQ data 200) and/or estimates from the estimator 260 (or a separate estimator) are input to the machine-learned segmentation model. For example, the processor 570 configures the filter 250 and estimator 260 to generate one or more sets of estimates for each location (e.g., velocity, variance, and/or power in one, two, three, or more versions corresponding to different frequency responses or filtering by the filter 250). In response to this input, the machine-learned segmentation model 242 outputs a segmentation map (discrimination map 244).

The image processor 570 uses the segmentation map to select the frequency response (i.e., program the programmable wall filter 250). Different frequency responses are used for different regions (i.e., types of signals identified by segmentation). The image processor 570 programs the wall filter 250, which then re-filters the scan data from the memory 540. This filtered scan data is used by the estimator 260 to estimate the velocity, variance, and/or power for each location.

The Doppler estimator 260 is a Doppler processor or cross-correlation processor for estimating the color data. In alternative embodiments, another device now known or later developed for estimating velocity, power (e.g., energy), and/or variance from any or various input data is provided. The estimator 260 receives a plurality of signals associated with a same location at different times and estimates a Doppler shift frequency, based on a change or an average change in phase between consecutive signals from the same location. Velocity is calculated from the Doppler shift frequency. Alternatively, the Doppler shift frequency is used as a velocity. The power and variance may also be calculated.

Color data (e.g., velocity, power, and/or variance) is estimated for spatial locations in the scan region from the filtered beamformed scan samples. For example, the color data represents a plurality of different locations in a plane. The color flow data is motion data for tissue and/or fluid.

The flow estimator 260 may apply one or more thresholds to identify sufficient motion information. For example, velocity and/or power thresholding for identifying velocities is used. The estimator 260 implements a threshold 270. Alternatively, the processor 570 implements the threshold 270. The image processor 570 may configure the threshold 270 to adapt based on the segmentation map. In alternative embodiments, a separate processor or filter applies thresholds.

The estimator 260 outputs frames of data (estimates) representing the scan region at different times. The beamformed samples for a given flow sample count are used to estimate for a time. A moving window with overlap of the data is used to estimate for other times. Velocities for each location at different times are output.

The image processor 570 or another component uses the motion values output after thresholding to generate an image. The frame of estimates (e.g., velocities) is scan converted and color mapped. The resulting color values are added to a B-mode image, such as an overlay, or used alone. The color values are placed in a display buffer to display an image on the display 590.

The display 590 is a CRT, LCD, plasma, projector, monitor, printer, touch screen, or other now known or later developed display device. The display 590 receives RGB, other color values, or other motion values and outputs an image. The image may be a gray scale or color image. The image represents the region of the patient with greater diagnostic information content due to the filtering and/or threshold adaptation based on segmenting by the artificial intelligence. The display 590 displays a Doppler or other color image from the motion (color) values.

The memory 580 is video random-access memory, random access memory, removable media (e.g., diskette or compact disc), hard drive, database, or other memory device for storing color or other motion data. The stored data is in a polar or Cartesian coordinate format. The machine-learned segmentation model 242 may be stored in the memory 580, or a different memory. Settings for the programmable filter 250 and/or thresholds for the threshold 270 may be stored in the memory 580. The memory 580 is used by the image processor 570 for adapting the clutter filtering and/or thresholding.

The instructions for implementing the processes, methods and/or techniques discussed above are provided on computer-readable storage media or memories, such as a cache, buffer, RAM, removable media, hard drive, or other computer readable storage media. The memory 580 or other memory stores the instructions. Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing and the like. In some embodiments, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

Listed below are various Illustrative Embodiments. The Illustrative Embodiments summarize different combinations of aspects. Other combinations of any of the aspects with any other one or more of the aspects may be provided. Aspects from one type (e.g., method or system) may be used in another type (system or method).

Illustrative Embodiment 1. A method for adaptive clutter filtering in color imaging by an ultrasound scanner, the method comprising: scanning, by the ultrasound scanner, a patient; discriminating, by a machine-learned model, a first region from a second region by first and second types of signals represented in scan data from the scanning; applying a first wall filter for the first region of the first type of signal and a second wall filter for the second region of the second type of signal, the first wall filter different than the second wall filter; and color imaging, by the ultrasound scanner, using estimates resulting from the applying of the first and second wall filters.

Illustrative Embodiment 2. The method of Illustrative Embodiment 1 wherein applying comprises applying to the scan data, and wherein color imaging comprises color imaging from estimates of the scan data for the first region as filtered by the first wall filter and of the scan data for the second region as filtered by the second wall filter.

Illustrative Embodiment 3. The method of any of Illustrative Embodiments 1-2 wherein color imaging comprises color flow imaging where the first and second wall filters comprise high pass filters with different frequency responses.

Illustrative Embodiment 4. The method of any of Illustrative Embodiments 1-3 wherein discriminating comprises segmenting the first region from the second region sample location-by-sample location.

Illustrative Embodiment 5. The method of any of Illustrative Embodiments 1-4 wherein discriminating comprises discriminating by the first type of signal, the second type of signal, and a third type of signal, the third type of signal being at a third region, wherein applying comprises applying a third wall filter for the third region, the third wall filter different than the first and second wall filters, and wherein color imaging comprises color imaging using estimates resulting from applying of the third wall filter.

Illustrative Embodiment 6. The method of Illustrative Embodiment 5 wherein the first type of signal comprises flow signal from fluid or moving tissue signal from tissue, the second type of signal comprises flash and/or clutter, and the third type of signal comprises background noise, and wherein color imaging comprises color imaging of the fluid or moving tissue.

Illustrative Embodiment 7. The method of Illustrative Embodiment 6 wherein applying comprise applying with the first wall filter has a lowest cutoff frequency relative to the first, second, and third wall filters, the second wall filter has a highest cutoff frequency relative to the first, second, and third wall filters, and the third wall filter has a cutoff frequency between the highest and lowest cutoff frequencies relative to the first, second, and third wall filters.

Illustrative Embodiment 8. The method of any of Illustrative Embodiments 1-7 further comprising setting a threshold for the estimates for the first region differently than a threshold for the estimates for the second region, wherein the estimates for color imaging result from thresholding using the thresholds for the first and second regions.

Illustrative Embodiment 9. The method of any of Illustrative Embodiments 1-8 wherein discriminating comprises discriminating by the machine-learned model comprising a semantic segmentation deep network.

Illustrative Embodiment 10. The method of Illustrative Embodiment 9, wherein discriminating comprises discriminating by the semantic segmentation deep network comprising an image-to-image neural network.

Illustrative Embodiment 11. The method of any of Illustrative Embodiments 1-10, wherein discriminating comprises estimating velocity, variance, and/or power from the scan data and segmenting, by the machine-learned model, in response to input of the velocity, variance, and/or power to the machine-learned model.

Illustrative Embodiment 12. The method of Illustrative Embodiment 11, wherein estimating comprises estimating two or more versions of the velocity, variance, and/or power, and wherein the two or more versions are input to the machine-learned model.

Illustrative Embodiment 13. The method of any of Illustrative Embodiments 1-12 wherein color imaging comprises color imaging for one of various imaging applications, and wherein discriminating comprises discriminating by the machine-learned model being used for any of the various imaging applications.

Illustrative Embodiment 14. A method for adaptive clutter filtering in color imaging by an ultrasound scanner, the method comprising: generating a discrimination map discriminating sample locations into multiple categories, the discrimination map generated by an artificial intelligence; adapting clutter filtering based on the discrimination map; and color flow imaging using the clutter filtering as adapted.

Illustrative Embodiment 15. The method of Illustrative Embodiment 14 wherein generating comprises generating by the artificial intelligence in response to input of estimates of velocity, variance, and/or power to the artificial intelligence.

Illustrative Embodiment 16. The method of Illustrative Embodiment 15 wherein generating comprises generating by the artificial intelligence in response to input of multiple versions of the estimates for each of the sample locations, the multiple versions for each of the sample locations having different wall filtering.

Illustrative Embodiment 17. The method of any of Illustrative Embodiments 14-16 wherein generating comprises generating the discrimination map distinguishing between the sample locations with flow signal, the sample locations with clutter and/or flash, and the sample locations with background noise, and wherein adapting the clutter filtering comprises selecting different high pass frequency responses for the flow signal, clutter and/or flash, and background noise.

Illustrative Embodiment 18. An ultrasound system for color imaging, the ultrasound system comprising: a transducer and beamformer for scanning a scan region; a programmable wall filter; an image processor configured to segment, by application of a machine-learned segmentation model, the scan region into at least two classes and adapt settings of the programmable wall filter based on the at least two classes such that different locations of the scan region use different ones of the settings; a Doppler estimator configured to estimate, from data filtered by the programmable wall filter based on the settings, color values in the scan region; and a display configured to display an image using the color values.

Illustrative Embodiment 19. The ultrasound system of Illustrative Embodiment 18 wherein the image processor is configured to input estimates of velocity, variance, and/or power to the machine-learned segmentation model, the machine-learned segmentation model outputting a segmentation map in response to the input.

Illustrative Embodiment 20. The ultrasound system of Illustrative Embodiment 19 wherein the image processor is configured to input the estimates in multiple versions corresponding to different frequency response.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for adaptive clutter filtering in color imaging (140) by an ultrasound scanner (500), the method comprising:
scanning (100), by the ultrasound scanner (500), a patient;
discriminating (110), by a machine-learned model (242), a first region from a second region by first and second types of signals represented in scan data from the scanning (100);
applying (120) a first wall filter (250) for the first region of the first type of signal and a second wall filter (250) for the second region of the second type of signal, the first wall filter (250) different than the second wall filter (250); and
color imaging (140), by the ultrasound scanner (500), using estimates resulting from the applying (120) of the first and second wall filters (250).

2. The method of claim 1 wherein applying (120) comprises applying (120) to the scan data, and wherein color imaging (140) comprises color imaging (140) from estimates of the scan data for the first region as filtered by the first wall filter (250) and of the scan data for the second region as filtered by the second wall filter (250).

3. The method of claim 1 or 2 wherein color imaging (140) comprises color flow imaging (140) where the first and second wall filters (250) comprise high pass filters with different frequency responses.

4. The method of one of claims 1 to 3 wherein discriminating (110) comprises segmenting the first region from the second region sample location-by-sample location, and/or wherein discriminating (110) comprises discriminating (110) by the first type of signal, the second type of signal, and a third type of signal, the third type of signal being at a third region, wherein applying (120) comprises applying (120) a third wall filter (250) for the third region, the third wall filter (250) different than the first and second wall filters (250), and wherein color imaging (140) comprises color imaging (140) using estimates resulting from applying (120) of the third wall filter (250).

5. The method of claim 4 wherein the first type of signal comprises flow signal from fluid or moving tissue signal from tissue, the second type of signal comprises flash and/or clutter, and the third type of signal comprises background noise, and wherein color imaging (140) comprises color imaging (140) of the fluid or moving tissue.

6. The method of claim 5 wherein applying (120) comprises applying (120) with the first wall filter (250) has a lowest cutoff frequency relative to the first, second, and third wall filters (250), the second wall filter (250) has a highest cutoff frequency relative to the first, second, and third wall filters (250), and the third wall filter (250) has a cutoff frequency between the highest and lowest cutoff frequencies relative to the first, second, and third wall filters (250).

7. The method of one of claims 1 to 6 further comprising setting (130) a threshold for the estimates for the first region differently than a threshold for the estimates for the second region, wherein the estimates for color imaging (140) result from thresholding using the thresholds for the first and second regions.

8. The method of one of claims 1 to 7 wherein discriminating (110) comprises discriminating (110) by the machine-learned model (242) comprising a semantic segmentation deep network, wherein discriminating (110) particularly comprises discriminating (110) by the semantic segmentation deep network comprising an image-to-image neural network, and/or wherein discriminating (110) comprises estimating velocity, variance, and/or power from the scan data and segmenting, by the machine-learned model (242), in response to input of the velocity, variance, and/or power to the machine-learned model (242).

9. The method of claim 8, wherein estimating comprises estimating two or more versions of the velocity, variance, and/or power, and wherein the two or more versions are input to the machine-learned model (242).

10. The method of one of claims 1 to 9 wherein color imaging (140) comprises color imaging (140) for one of various imaging (140) applications, and wherein discriminating (110) comprises discriminating (110) by the machine-learned model (242) being used for any of the various imaging (140) applications.

11. A method for adaptive clutter filtering in color imaging (140) by an ultrasound scanner (500), the method comprising:
generating (112) a discrimination map (244) discriminating (110) sample locations into multiple categories, the discrimination map (244) generated by an artificial intelligence (242);
adapting (122) clutter filtering based on the discrimination map (244); and
color flow imaging (140) using the clutter filtering as adapted.

12. The method of claim 11 wherein generating (112) comprises generating (112) by the artificial intelligence (242) in response to input of estimates (240) of velocity, variance, and/or power to the artificial intelligence (242), wherein generating (112) particularly comprises generating (112) by the artificial intelligence (242) in response to input of multiple versions of the estimates (240) for each of the sample locations, the multiple versions for each of the sample locations having different wall filtering, and/or wherein generating (112) comprises generating (112) the discrimination map (244) distinguishing between the sample locations with flow signal, the sample locations with clutter and/or flash, and the sample locations with background noise, and wherein adapting (122) the clutter filtering comprises selecting different high pass frequency responses for the flow signal, clutter and/or flash, and background noise.

13. An ultrasound system for color imaging (140), the ultrasound system comprising:
a transducer (520) and beamformer for scanning (100) a scan region;
a programmable wall filter (250);
an image processor (570) configured to segment, by application of a machine-learned segmentation model (242), the scan region into at least two classes and adapt settings (130) of the programmable wall filter (250) based on the at least two classes such that different locations of the scan region use different ones of the settings (130);
a Doppler estimator (260) configured to estimate, from data filtered by the programmable wall filter (250) based on the setting (130)s, color values in the scan region; and
a display (590) configured to display an image using the color values.

14. The ultrasound system of claim 13 wherein the image processor (570) is configured to input estimates (240) of velocity, variance, and/or power to the machine-learned segmentation model (242), the machine-learned segmentation model (242) outputting a segmentation map in response to the input.

15. The ultrasound system of claim 13 or 14 wherein the image processor (570) is configured to input the estimates (240) in multiple versions corresponding to different frequency response.
